# EUROPEAN PATENT SPECIFICATION

(11) **EP 2 606 968 B1**
(45) Date of publication and mention of the grant of the patent: **20.01.2016**
(21) Application number: 12196379.7
(22) Date of filing: 10.12.2012
(51) Int. Cl.: C07C 51/235, C07C 59/08, B01J 21/18, B01J 23/42, B01J 35/00, B01J 35/10, B01J 37/02, B01J 37/08

(54) **Process for the preparation of lactic acid from glycerol**
Verfahren zur Herstellung von Milchsäure aus Glycerol
Procédé de préparation d'acide lactique à partir de glycérol

(30) Priority: 23.12.2011 LV 110174
(43) Date of publication of application: 26.06.2013
(73) Proprietor: Rigas Tehniska universitate, Riga 1658 (LV); RTU Neorganiskas Kimijas Instituts, RTU agentura, 2169 Salaspils (LV)
(72) Inventor: Cornaja, Svetlana, LV-1015 Riga (LV); Dubencovs, Konstantins, LV-1046 Riga (LV); Kulikova, Lidija, LV-1013 Riga (LV); Serga, Vera, LV-1005 Riga (LV); Kampars, Valdis, LV-1082 Riga (LV); Zizkuna, Svetlana, LV-1006 Riga (LV); Stepanova, Olga, LV-1035 Riga (LV); Sproge, Elina, LV-2125 Baldone (LV); Cvetkovs, Antons, LV-2010 Jurmala (LV)
(74) Representative: Fortuna, Jevgenijs

(56) References cited:
- EP-A1- 2 332 899
- CN-A- 101 695 657
- SHEN, Y. ET AL.: "Efficient synthesis of lactic acid by aerobic oxidation of glycerol on Au-Pt/TiO2 catalysts", CHEMISTRY - A EUROPEAN JOURNAL, vol. 16, no. 25, 18 May 2010 (2010-05-18), pages 7368-7371, XP009146561, ISSN: 0947-6539, DOI: 10.1002/CHEM.201000740 [retrieved on 2010-05-18]
- RAMÍREZ-LÓPEZ, C.A. ET AL.: "Synthesis of lactic acid by alkaline hydrothermal conversion of glycerol at high glycerol conversion", IND. ENG. CHEM. RES., vol. 49, no. 14, 2010, pages 6270-6278, XP55046853,
- PALCEVSKIS, E. ET AL.: "Catalyst materials based on plasma-processed alumina nanopowder", JOURNAL OF THE SERBIAN CHEMICAL SOCIETY, vol. 77, no. 12, December 2012 (2012-12), pages 1799-1806, XP55089868, ISSN: 0352-5139, DOI: 10.2298/JSC121116147P

## Description

### Technical field

The invention relates to chemical technology, particularly to production of lactic acid using selective oxidation of glycerol by molecular oxygen in presence of platinum supported heterogeneous catalysts.

### Background art

In biodiesel fuel manufacturing process transesterification of vegetable oil in an alkaline medium produces such a valuable by-product as glycerol. The yield of glycerol makes up about 10-14 weight % of overall mass of the products. Glycerol is a multifunctional substance. A number of important and valuable products used as raw products in various processes of organic synthesis can be obtained in the process of glycerol oxidation [1-3]. One of the possible products is lactic acid. The lactic acid is widely used in food manufacturing, pharmacy, cosmetics and so on [4-7]. The demand for lactic acid is increasing, in particular in recent years. From it an ecologically clean biodegradable polymer - polylactic acid is obtained [8, 9], which is also biologically compatible material for medical applications [4-7, 10].

Industrial lactic acid nowadays is produced from carbohydrates, using an expensive and time-consuming enzymatic method [9], characterized by low productivity or environmental hazards, as well as an expensive method based on the reaction of acetaldehyde with hydrogen cyanide followed by the hydrolysis of the lactonitrile [4-7,11]. Great attention during last years has been paid to the research of various methods of glycerol processing resulting in lactic acid [11-18].

A part of investigations is devoted to the hydrothermal reactions of glycerol with alkali [11-13], resulting in lactic acid, another part - to the investigation of glycerol catalytic hydrogenolysis [14, 15]. These two methods for obtaining lactic acid from glycerol have significant drawback: they require expensive equipment (autoclaves) and the process takes place at very high temperature 200-350°C. As the result of reaction in hydrothermal processes molecular hydrogen is released, increasing the pressure inside the reactor, but the glycerol hydrogenolysis takes place in reaction of glycerol and hydrogen at a pressure of 40 bar, this, from a technical point of view, makes the two processes very dangerous.

The use of process for catalytic oxidation of glycerol by molecular oxygen with the possibility of catalyst regeneration can make the process of obtaining lactic acid cheaper. It is also important that the oxidation process is environmentally clean (oxygen - ecologically clean oxidant, the solvent is water) and can be conducted at mild conditions (at low temperature, at the atmospheric pressure). For glycerol oxidation by molecular oxygen heterogeneous supported Pt, Pd and Au based catalysts with carbon based carriers are used. The metal oxide based carriers are used less frequently. The main problem in the investigation of glycerol oxidation by molecular oxygen is an active and selective catalyst synthesis to obtain one product from the possible products. Glycerol oxidation products are mostly obtained in a mixture.

Up to now according to the literature [19, 20] high selectivity and yield is achieved only after glyceric acid. By modifying the active metal nature of catalyst and methods of catalyst synthesis, the catalyst selectivity and the oxidation process' main product yield can be changed.Different methods for synthesis of catalysts are used when performing catalytic glycerol oxidation: metal sol immobilization method (active metal fixing on the carrier's surface) [21], impregnation or dry impregnation method (carrier impregnation with a metal salt aqueous solution, followed by its reduction) [18, 22, 23], deposition - precipitation method (active metal hydroxide precipitation, followed by its chemical or thermal reduction) [18,24], co-precipitation method (active metal and carrier hydroxide simultaneous precipitation, followed by its chemical or thermal reduction) [18], ion-exchange method [25], magnetron spraying method [26], extraction-pyrolytic method [27].

The best results in glycerol oxidation process resulting in lactic acid, was achieved in [16-18]. In [16] glycerol is oxidized in the presence of various gold-containing commercial catalysts. The resulting lactic acid yield varies in the range of 9.7 - 32 % at glycerol conversion range 70 - 12 %. In addition lactic acid yield selectivity varies in the range 33 - 80 %.

The closest to the applied method for obtaining lactic acid are the results of [18]. In the closest prior art glycerol is oxidized by molecular oxygen in the presence of supported Pt-containing catalysts, resulting in lactic acid as a basic product. The authors of [18] synthesize and use for oxidation process one mono-metallic Pt(0.25-0.75%)/TiO₂, as well as mixed catalysts: Pt(0.25-0.75%) - Pd(0.5-1.0%)/ZrO₂; Pt(0.25-0.75%) - Pd(0.5-1.0%)/C; Pt(0.25-0.75%) - Rh(0.5-1.5%)/Fe₂O₃; Pt(0.25-0.75%) - Au(1.25-1.75%)/CeO₂; Pt(0.25-0.75%) - Ru(1.25-1.75%) - Rh(0.5-1.5%)/Al₂O₃. In the closest prior art solution catalysts are synthesized from inorganic precursors by dry impregnation (Pt-Pd/ZrO₂; Pt-Pd/C), wet impregnation (Pt/TiO₂, Pt-Ru-Rh/Al₂O₃), co-precipitation (Pt-Rh/Fe₂O₃) or deposition - precipitation (Pt- Au/CeO₂) method. The carriers using impregnation method are impregnated by acid catalyst precursor solutions at 20 - 40 °C temperature. After the carrier impregnation by dry impregnation method metals are reduced in the molecular hydrogen atmosphere at 150 - 290 °C temperature for 2 - 7 hours. After the carrier impregnation by wet impregnation method metals are reduced by NaBH₄ (Pt-Ru-Rh/Al₂O₃) or by formaldehyde (Pt/TiO₂) solution for 4 - 6 hours, after the reduction the catalysts are being filtered, washed and dried at 100 - 120°C temperature for 15 - 17 hours. When supported catalysts are synthesized by co-precipitation method, carriers and supported active metal hydroxides simultaneously are obtained from the mixture of precursor solution by its treatment with sodium hydroxide solution. Then residue is washed and active metal hydroxides are reduced at hydroxide atmosphere for 3 - 5 hours at 300 - 400°C temperature. Using catalyst precipitation synthesizing method catalyst carrier is impregnated by precursor solutions, after the impregnation sodium hydroxide solution is added to suspension and after stirring during 10 - 15 hours the suspension obtained is filtered, washed and reduced at hydroxide atmosphere during 5 - 7 hours at 270 - 290°C temperature. The glycerol alkaline water solution is being oxidised in the thermostated barbotage-type glass reactor or in autoclave at pressure of 101325-506625 Pa (1-5 atm) within the range of temperature 0-140 °C.

The major deficiencies of all the methods for catalyst synthesis being used in the closest prior art are given below: (i) for the preparation of precursors and chemical reducing agents specially pure reagents are used; (ii) in wet impregnation method harmful (formaldehyde) or dangerous (sodium tetra-boron-hydride) substances are used; (iii) in dry impregnation, co-precipitation and precipitation methods for the catalyst precursor reduction expensive and explosive molecular hydrogen at very high temperature (150 - 400 °C) is used; (iv) in wet impregnation, co-precipitation and precipitations methods the use of solutions of catalysts inorganic precursors and reducing agents (formaldehyde and NaBH4) leads to formation of large volumes of hazardous waste water, that requires great expenses for its utilization; (v) in co-precipitation and precipitation methods rather strict control of process parameters is needed (temperature, stirring speed, mixture pH control) for obtaining of active catalysts of good quality.

### Disclosure of Invention

Theessence of the invention is to develop a new method for platinum-containingcatalysts synthesis, which provides selective and active catalysts synthesis for obtaining lactic acid using glycerol oxidation by molecular oxygen in constructively simple equipment in mild conditions. The main aim of the invention is to develop simpler, cheaper and more environmentally friendly platinum-containing catalyst synthesis process. The proposed process provides obtaining of active and selective catalysts by extraction-pyrolytic method. In [27] the extraction-pyrolytic method was used for supported palladium-containing catalysts synthesis for glyceric acids production from glycerol. The method proposed according to the invention differs from the catalysts obtaining process described in [27] by platinum as the active component of catalysts, as well as by the catalysts carriers composition. Except Al₂O₃, Y₂O₃ there are different, the new platinum catalysts being selective by lactic acid, not by glyceric acid as it is in [27]. The use of new supported platinum-containing catalysts provides selective lactic acid production with great yield, which is ecologically cleaner, of general-purpose and cheaper than that one in the closest prior art solution, using constructively simple oxidation equipment of different types. The supported platinum catalysts obtained according to the invention differ from that of the closest prior art either by the composition of active metals (in the closest prior art basically composite Pt, Pd, Rh and Ru containing catalysts are synthesized, the only mono-metallic catalyst is Pt/TiO₂) or by the composition of the carrier in case of mono-metallic catalysts. Unlike the closest prior art in the invention all catalysts are mono-metallic platinum-containing catalysts, wherein Al₂O₃, Y₂O₃, C and ZrO₂ nano-powders with high specific surface area (20 - 337 m²/g) as carriers are used. Catalyst synthesis in the invention, unlike the closest prior art, starts with platinum compounds extraction from hydrochloric acid solution with trioctylamine ((C₈H₁₇)₃N) solution in toluene, obtaining platinum-containing precursors. Especially clean raw materials are not required for obtaining of precursor; purification of platinum from impurities is performed during the extraction step. After the extraction step determined volume of organic precursor is added to the weighed amount of the carrier. After carrier impregnation with precursor the mixture obtained is being dried for 5 - 180 min at 20 - 100°C temperature. Unlike in the closest prior art chemical reducing agents are not required for the platinum reduction.

The proposed method for obtaining lactic acid from glycerol comprises the following steps: i) obtaining of mono-metallic supported platinum-containing catalyst by following consecutive steps: a) obtaining platinum-containing precursor by extraction of platinum compound from hydrochloric acid solution with trioctylamine ((C₈H₁₇)₃N) solution in toluene, b) carrier impregnation with the platinum-containing organic precursor solution obtained in the previous step, c) drying of the impregnated carrier, d) platinum pyrolytic reduction; ii) glycerol oxidation by molecular oxygen in the presence of platinum-containing supported catalysts in alkaline aqueous solutions.

Platinum reduction to the metal occurs during pyrolysis of dry mixture of carrier with precursor at 300-500°C temperature for 5 - 120 minutes at the atmospheric pressure. During pyrolysis the complete decomposition of organics component of the mixture occurs, as a result pure catalyst is being obtained after pyrolysis and, unlike in the closest prior art, the washing and drying steps are excluded, and metal losses are prevented as well. The supported platinum catalysts with highly dispersed platinum particles with a size of 6 - 35 nm are obtained. Platinum content in catalyst is in the range between 0.6 and 5.0 wt%. The examples of catalysts obtained and their characteristics are given in Table 1.

**Table 1 Examples of the new supported platinum catalysts**

| Catalyst | t°_{pyr}, °C | t, min | SSA sup. m²/g | SSA catal. m²/g | d_{cryst}. nm |
|---|---|---|---|---|---|
| 1,25wt%Pt/ZrO₂ | 300 | 5 | 65 | 59.5 | 8-9 |
| 5,0%wtPt/Y₂O₃ | 300 | 5 | 20 | | 6 |
| 2,5%wtPt/Al₂O₃ | 500 | 5 | 41 | 40 | 20 |
| 5,0wt%Pt/ C | 300 | 120 | 337 | 150 | 35 |

| | | | | | |
|---|---|---|---|---|---|
| t°_{pyr}- pyrolysis temperature; t - time of pyrolysis; SSA sup. - the specific surface area of carrier; SSA catal.- the specific surface area of catalyst; d_{cryst} - the average size of platinum crystallites | | | | | |

The lactic acid according to the invention is being obtained using the process of glycerol oxidation by molecular oxygen in the presence of supported platinum catalysts in alkaline aqueous solutions at 50 - 70°C temperature at the atmospheric pressure in the barbotage-type reactor without additional stirring or in closed type batch reactor with magnetic mixer. Glycerol oxidation conditions are the following: P_{O₂}= 20265-101325 Pa (0.2-1.0 atm); c₀(glyc) = 0.3 - 1.0 mol/L (M); c₀(NaOH) = 0.7 - 2.0 mol/L (M); n(glyc)/n(Pt) = 200 - 500 mol/mol. Time of glycerol oxidation to lactic acid depending on the conditions of the process is 4 - 12 hours.

The new supported platinum containing catalysts obtained according to the invention are active and selective in all the examined conditions. The selectivity of the lactic acid yield varies in the range of 34 - 70% at the glycerol conversion rate 36 - 87% depending on the composition of the catalyst and oxidation time (Table 2).

**Table 2**

| Catalytic oxidation of glycerol by molecular oxygen in the presence of new supported Pt catalysts c₀(glyc) = 0.3 mol/L (M); c₀(NaOH) = 1.5 mol/L (M); n(glyc)/n(Pt) = 300 mol/mol (^{a} 400 mol/mol); t = 60°C (^{b} 65°C); | | | | |
|---|---|---|---|---|
| Catalyst | Po₂, 10⁻⁵ Pa | Oxidation time, hours | Glycerol conversion, % | Selectivity to lactic acid, % |
| 1.25wt%Pt/ZrO₂ | 1.0 | 7 | 87 | 34 |
| 0.6% wtPt/Y₂O₃ | 1.0 | 4 | 67 | 42 |
| 1.25%wt Pt/Y₂O₃ | 1.0 | 4 | 86 | 63 |
| 2.5%wtPt/Y₂O₃ | 1.0 | 4 | 55 | 52 |
| 1.25%wtPt/Al₂O₃ | 1.0 | 4 | 54 | 60 |
| | 0.2 | 7 | 49 | 60 |
| 2.5%wtPt/Al₂O₃ | 1.0 | 4 | 49 | 61 |
| | 0.2 | 7 | 45 | 60 |
| | 0.2 | 7 | 36^{a} | 66 |
| | 0.2 | 7 | 37^{b} | 70 |
| 5%wtPt/C | 1.0 | 4 | 65 | 41 |

| | | | | |
|---|---|---|---|---|
| By-products: glyceric acid, tartronic acid, glycolic acid, oxalic acid. | | | | |

The deficiencies of platinum supported catalysts obtaining methods used in the closest prior art are eliminated and the aim or the invention is achieved by synthesizing the catalysts required obtaining for lactic acid from glycerol using the offered extraction-pyrolytic method. The proposed method is much simpler, cheaper and more environmentally friendly than the closest prior art solution. The characteristics and advantages of the proposed method are as follows: (i) especially clean raw materials are not necessary for obtaining catalysts organic precursors - platinum purification from impurity is performed during extraction step; consequently reuse of platinum-based technological solutions and secondary raw materials is possible; (ii) metal losses are prevented during carrier impregnation and metal reduction steps; (iii) the use of chemical reducing agents is not required, because platinum reduction occurs during the pyrolysis; (iv) the method allows to modify and optimize composition of catalysts, structure and properties in wide range for the selective lactic acid obtaining using oxidation of glycerol by molecular oxygen; (v) the method allows to simplify catalysts and lactic acid production technology and make it cheaper, and to comply with environmental requirements as well.

The supported platinum-containings catalysts are obtained by extraction-pyrolytic method as follows: a carrier is impregnated with organic precursor solution, obtained by extraction of platinum from hydrochloric acid solution with trioctylamine solution in toluene; after the carrier impregnation with precursor the mixture obtained is being dried and catalyst's metal is thermal reduced at 300 - 500 °C temperature. Pure supported platinum containing catalysts with nanoscale platinum particles (6 - 35 nm) are obtained.

For better understanding of the invention, the examples of catalyst preparation and lactic acid production are offered.

**Example 1.** Preparation of catalyst precursor.38.7 mL 0.5 mol/L (M) platinum hydrochloric acid H₂PtCl₆ solution in 2 mol/L (M) hydrochloric acid is being added to 48.25 mL 1 mol/L (M) trioctylamine solution in toluene. After extraction of mixture during 3 minutes, the organic phase is being separated from the inorganic one and filtered through the paper filter. The platinum compound [(C₈H₁₇)NH]₂PtCl₆ is being created during extraction of organic precursor in solution.

**Example 2.** Synthesis of 2.5 wt% Pt/Al₂O₃ catalyst. 1.0 g Al₂O₃ nanopowder is added to 0.32 mL precursor solution (precursor concentration - 0.4 mol/L (M)). The system obtained is being stirred up to carrier impregnation by precursor and is being dried for 5 min at 80 - 98 °C temperature. After drying the dry mixture is being heated up to 400 °C at the speed 10 degr./min and calcinated at 400°C temperature for 5 minutes at the atmospheric pressure. Pure 2.5 wt%Pt/Al₂O₃ catalyst is being obtained with d_{cryst}= 16 nm. The yield of 2.5 wt%Pt/Al₂O₃ catalyst is 1.0 g.

**Example 3.** Synthesis of 1.25 wt% Pt/Al₂O₃ catalyst. Analogous to example 2,but the difference is that the volume of precursor solution is 0.16 mL and drying time at 80-86°C temperature is 3 minutes. Pure 1.25 wt%Pt/Al₂O₃ catalyst is being obtained. 1.25 wt% Pt/Al₂O₃ catalyst yield is 1.0 g.

**Example 4.** Synthesis of 2.5 wt% Pt/Y₂O₃ catalyst. 1.0 g Y₂O₃ powder is added 0.32 mL precursor solution (precursor concentration - 0.4 mol/L (M). The system obtained is being stirred up to carrier impregnation by precursor and is being dried for 5 min at 80 - 98 °C temperature. After drying the dry mixture is being heated up to 400 °C at the speed 10 degr./min and calcinated at 400°C temperature for 5 minutes at the atmospheric pressure. Pure 2.5 wt%Pt/Al₂O₃ catalyst is being obtained with yield 1.0 g.

**Example 5.** Synthesis of 5 wt%Pt/Ccatalyst. 1.0 g C powder is added 2 mL precursor solution (precursor concentration 0.128 mol/L(M). The system obtained is being stirred up to carrier impregnation by precursor and is being dried for 120 min at 20°C temperature. The dry mixture is being heated up to 300 °C at the speed 10 degr./min and calcinated at 300 °C temperature for 120 minutes at the atmospheric pressure. Pure 5 wt%Pt/C catalyst with SSA= 150 m²/g and d_{cryst} = 35 nm is being obtained. 5 wt%Pt/C catalyst yield is 1.0 g.

**Example 6.** The process for glycerol oxidation in barbotage type reactor. Conditions of oxidation: P_{O₂} = 101325 Pa (1 atm); 60°C; c₀(glyc) = 0.3 mol/L (M); c₀(NaOH) = 1.5 mol/L (M); n(glyc)/n(Pt) = 300 mol/mol. The total volume of the reactor is 50 mL, the total volume of oxidate is 15 mL. The oxidation is being performed as follows: during thermostating (10 min) 0.117 g of dry 2.5 wt% Pt/Al₂O₃ catalyst, 9.5 mL distilled water and 1.0 mL 4.5 mol/L (M) glycerol aqueous solution are being introduced to the reactor. The supply of oxygen to reactor is being switched on and 4.5 mL 5.0 mol/L (M) sodium hydroxide aqueous solution is being added. The oxidation process starts at the moment of addition of sodium hydroxide. The speed of oxygen flow is 300 mL/min. In order to define the concentration of products of reaction, samples are taken every 60 minutes. Samples of oxydates are being filtered for separation of catalysts. The filtered sample is being acidized by 9.0 mol/L (M) sulfuric acid solution up to pH = 2 - 3 and and analyzed by high performance liquid chromatographic (HPLC) method. After 7 hours glycerol conversion is 56%; selectivity of lactic acid production - 57 %. The selectivity of by-products production is: glyceric acid - 6 %, tartronic acid - 26 %.

**Example 7.** Analogous to Example 6, but the difference is that 2.5 wt% Pt/Y₂O₃ catalyst is being used and that 0.117 g of dry 2.5 wt%Pt/Y₂O₃ catalyst is being fed to the reactor. After 7 hours the conversion of glycerol is 59 %; selectivity of lactic acid production - 49 %. The selectivity of by-products production is: glyceric acid - 12 %, tartronic acid - 29 %.

**Example 8.** Analogous to Example 6, but the difference is that 1.25 wt% Pt/ZrO₂ catalyst is being used and 0.234 g of dry 1.25 wt%Pt/ZrO₂ catalyst is being fed to the reactor. After 7 hours the conversion of glycerol is 87 %; selectivity of lactic acid production - 34 %. The selectivity of by-products production is: glyceric acid - 20 %, tartronic acid - 31 %.

**Example 9.** Analogous to Example 6, but the difference is that 5 wt%Pt/C catalyst is being used and 0.0585 g of dry 5 wt%Pt/C catalyst is being fed to the reactor. After 4 hours the conversion of glycerol is 65 %; selectivity of lactic acid production - 41 %. The selectivity of by-products production is: glyceric acid - 21 %, tartronic acid - 24 %.

### Industrial applicability of the invention

The proposed invention can be used for the industrial preparation of active and selective supported platinum-containing catalyst synthesis, and of valuable glycerol oxidation product - lactic acid - as well.

The processes for the catalyst synthesis and lactic acid production are economically beneficial and ecologically clean. The method of lactic acid production is one of the possible ways of utilization of glycerol, obtained as bio-diesel fuel production process' by-product.

The invention provides new active and selective catalysts synthesis using extraction-pyrolytic method for lactic acid production, by catalytic oxidation of glycerol by molecular oxygen in alkaline aqueous solutions. Lactic acid is used in food manufacturing, pharmacy, cosmetics. Lactic acid is being used for ecologically pure biodegradable polymer - polilactic acid production, which is also biologically compatible material for medical applications [4-9].

### Sources of information

[1] *Pat.* US 6630130 (07.10.2003).
[2] *Pat.* JP 04120008 (21.04.1992).
[3] E.Chiellini. Polyesters Based on Glyceric Acid Derivatives as Potential Biodegradable Materials. J. Bioact. Compact. Polym., 1990, 5(1), 16 - 30.
[4] Y.Fan, C.Zhou, X.Zhu. Selective catalysis of lactic acid to produce commodity chemicals. Catal. Rev. Sci. Eng., 2009, 51, 293-324.
[5] R.M.West, M.S.Holm, S.Saravanamurugan, J.Xiong, Z.Beversdorf, E.Taarning, C.H.Christensen. Zeolite H-USY for the production of lactic acid and methyl lactate from C3-sugars.J. Catal., 2010, 269, 122-130.
[6] J.Lunt. Large-scale production, properties and commercial applications of polylactic acid polymers. Polym. Degrad. Stab., 1998, 59(1-3), 145-152.
[7] K.Fukushima, Y.Kimura. Stereocomplexed polylactides (Neo-PLA) as high-performance bio-based polymers: their formation, properties, and application. Polim. Int., 2006, 55, 626-642.
[8] *Pat.* US 5543494 (08.06.1996).
[9] *Pat.* CA 2748354 (01.07.2010).
[10] C.X.F.Lam, R.Olkowski, W.Swieszkowski, K.C.Tan, I.Gibson, D.W.Hutmacher. Mechanical and in vitro evaluations of composite PLDLLA/TCP scaffolds for bone engineering. Virtual and Physical Prototyping, 2008, 3(4), 193-197.
[11] *Pat.* US 7829740 (9.11.2010).
[12] H.Kishida, F.Jin, Z.Zhou, T.Moriya, H.Enomoto. Conversion of glycerin into lactic acid by alkaline hydrothermal reaction.Chem. Lett., 2005, 34(11),1560.
[13] Z.Shen, F.Jin, Y.Zhang, B.Wu, A.Kishita, K.Tohji, H.Kishida. Effect of alkaline catalysts on hydrothermal conversion of glycerin into lactic acid. Ind. Erg. Chem. Res., 2009, 48(19), 8920-8925.
[14] E.P.Maris, R.J.Davis. Hydrogenolysis of glycerol over carbon-supported Ru and Pt catalysts. J. Catal., 2007, 249, 328-337.
[15]J.Dam, F.Kapteijn, K.Djanashvili, U.Hanefeld. Tuning selectivity of Pt/CaCO3 in glycerol hydrogenolysis - A Design of Experiments approach. Catal. Com. 2011, 13, 1-5.
[16] *Pat.* CN 101225041 (23.07.2008).
[17] Y.Shen, S.Zhang, H.Li, Y.Ren, H.Liu. Efficient synthesis of lactic acid by aerobic oxidation of glycerol on Au-Pt/TiO2 catalysts. Chem. Eur. J., 2010, 16, 7368 - 7371.
[18] *Pat.* CN 101695657 (21.04.2010).
[19] R.Garcia, M.Besson. P.Gallezot. Chemoselective catalytic oxidation of glycerol with air on platinum metals. Appl. Catal.: A, 1995, 127, 165-176.
[20] F.Porta, L.Prati. Selective oxidation of glycerol to sodium glycerate with gold-on-carbon catalyst an insight into reaction selectivity. J. Catal., 2004, 224, 397-403.
[21] A.Villa, D.Wang, D.S.Su, L.Prati. Gold Sols as Catalysts for Glycerol Oxidation: The Role of Stabilizer. Chem. Cat. Chem., 2009, 1, 510-514.
[22] S.D.Pollington, D.I.Enache, P.Landon, S.Meenakshisundaram, N.Dimitratos, A.Wagland, G.J.Hutchings, E.H.Stitt. Enhanced selective glycerol oxidation in multiphase structured reactors. Catal. Today, 2009, 145(1-2), 169-175.
[23] D.Liang, J.Gao, J.Wang, P.Chen, Y.Wei, Z.Hou, Bimetallic Pt-Cu catalysts for glycerol oxidation with oxygen in a base-free aqueous solution. Catal. Commun., 2011, 12, 1059-1062.
[24] N.Dimitratos, A.Villa, C.L.Bianchi, L.Prati, M.Makkee. Gold on titania: Effect of preparation method in the liquid phase oxidation. Appl. Catal: A, 2006, 51, 185-192.
[25] J.Gao, D.Liang, P.Chen, Z,Hou , X.Zheng. Oxidation of Glycerol with Oxygen in a Base-free Aqueous Solution over Pt/AC and Pt/MWNTs Catalysts. Catal. Lett., 2009, 130, 185-191.
[26] G.M.Veith, A.R.Lupini a, S.J.Pennycook, A.Villa, L.Prati, N.J.Dudney. Magnetron sputtering of gold nanoparticles onto WO3 and activated carbon. Catal. Today, 2007, 122, 248-253.
[27] *Pat.* LV 14079B (20.05.2010.)

## Claims

1. A method for obtaining lactic acid from glycerol, comprising the following steps:
i) obtaining of supported mono-metallic platinum containing catalyst by following consecutive steps:
a) obtaining of platinum-containing organic precursor by extraction of platinum compound from hydrochloric acid solution with trioctylamine ((C₈H₁₇)₃N) solution in toluene, wherein the concentration of platinum containing precursor solution is in the range between 0.03 and 0.4 mol/L(M),
b) impregnation of carrier with platinum-containing organic precursor solution obtained in the previous step, wherein the carrier is selected from the group consisting of Al₂O₃, Y₂O₃, C or ZrO₂ nanopowders with surface area between 20 and 337 m²/g,
c) drying of the impregnated carrierat temperature in the range between 20 and 100 °C during time interval between 3 and 120 minutes,
d) pyrolytic reduction of platinum;
ii) glycerol oxidation by molecular oxygen in the presence of supported platinum-containing catalysts in alkaline aqueous solution, wherein Pt content in catalysts is in the range between 0.6 wt% and 5.0 wt%.

2. The method according to claim 1, wherein glycerol oxidation is being performed at the temperature from 50 to 70 °C at the atmospheric pressure.

3. The method according to claim 1, wherein glycerol oxidation is being performed at the temperature from 50 to 70 °C at pressure between 20265 and 101325 Pa (between 0.2 and 1.0 atm), the initial molar concentration of glycerol c₀(glyc) is selected in the range between 0.3 and 1.0 mol/L (M); the initial molar concentration of sodium hydroxide c₀(NaOH) is selected in the range between 0.7 and 2.0 mol/L (M), the molar ratio of glycerol and platinum n(glyc)/n(Pt) is between 200 and 500 mol/mol.

4. The method according to any of the preceding claims, wherein carrier impregnation time is selected in the range between 5 and 120 minutes.

5. The method according to any of the preceding claims, wherein pyrolysis is performed at temperature in the range between 300 and 500 °C for 5 - 120 minutes at atmospheric pressure.

## Patentansprüche

1. Verfahren zur Herstellung von Milchsäure aus Glyzerin, die folgende Schritte umfasst:
i) Herstellung von unterstütztem monometallischen Platins, das einen Katalysator enthält, in folgenden Schritten:
a) Gewinnung einer platinhaltigen organischen Vorstufe durch Extraktion der Platinverbindung aus einer Salzsäurelösung mit einer Trioctylaminlösung ((C₈H₁₇)₃N) n Toluol, wobei die Konzentration der platinhaltigen Vorläuferlösung im Bereich zwischen 0,03 und 0,4 mol/L(M) liegt,
b) Imprägnierung der in der vorherigen Stufe hergestellten platinhaltigen organischen Vorläuferlösung, wobei ein Träger ausgewählt wird der aus der Gruppe Al₂O₃, Y₂O₃, C oder ZrO₂ Nanopulver besteht und eine Fläche zwischen 20 und 337 m²/g hat,
c) Trocknung des imprägnierten Trägers bei einer Temperatur im Bereich zwischen 20 und 100 °C bei Zeitintervallen von 3 und 120 Minuten,
d) pyrolytische Reduktion des Platins;
ii) Glycerinoxidation durch molekularen Sauerstoff in Gegenwart eines trägergebundenen platinhaltigen Katalysators in alkalisch wässriger Lösung, wobei der Pt-Gehalt in den Katalysatoren im Bereich zwischen 0,6 Gewichts-% und 5,0 Gewichts-% liegt.

2. Verfahren gemäß Anspruch 1, wobei die Glyzerinoxidation bei einer Temperatur von 50 bis 70 °C bei Atmosphärendruck herbeigeführt wird.

3. Verfahren gemäß Anspruch 1, wobei die Glyzerinoxidation bei einer Temperatur zwischen 50 und 70 °C bei einem Druck zwischen 20265 und 101325 Pa (zwischen 0,2 und 1,0 atm) stattfindet, wird die anfängliche molare Glyzerinkonzentration c₀(glyc) in einem Bereich von 0,3 und 1,0 mol/L (M) ausgewählt; die anfängliche molare Konzentration des Natriumhydroxid c₀(NaOH) wird in einem Bereich von 0,7 und 2,0 mol/L (M) ausgewählt, das Molverhältnis von Glyzerin und Platin n(glyc)/n(Pt) liegt zwischen 200 und 500 mol/mol.

4. Verfahren gemäß einem der vorangehenden Ansprüche, wobei eine Trägerimprägnierungszeit zwischen 5 und 120 Minuten gewählt wird.

5. Verfahren gemäß einem der vorangehenden Ansprüche, wobei die Pryolse bei einer Temperatur zwischen 300 und 500 °C während 5 - 120 Minuten bei Atmosphärendruck stattfindet.

## Revendications

1. Procédé d'obtention d'acide lactique à partir de glycérol, comprenant les étapes suivantes :
i) obtention d'un catalyseur supporté contenant du platine monométallique par les étapes consécutives suivantes :
a) obtention d'un précurseur organique contenant du platine par extraction du composé platine à partir d'une solution d'acide chlorhydrique avec une solution de trioctylamine ((C₈H₁₇)₃N) dans du toluène, dans lequel la concentration de la solution de précurseur organique contenant du platine est dans la plage comprise entre 0,03 et 0,4 mol/L(M),
b) imprégnation d'un support avec la solution de précurseur organique contenant du platine obtenue à l'étape précédente, dans lequel le support est choisi dans le groupe constitué de nanopoudres d'Al₂O₃, Y₂O₃, C ou ZrO₂ avec une surface spécifique comprise entre 20 et 337 m²/g,
c) séchage du support imprégné à une température dans la plage comprise entre 20 et 100 °C pendant un intervalle de temps compris entre 3 et 120 minutes,
d) réduction pyrolytique du platine ;
ii) oxydation du glycérol par de l'oxygène moléculaire en présence de catalyseurs supportés contenant du platine dans une solution aqueuse alcaline, dans lequel la teneur en Pt dans les catalyseurs est dans la plage comprise entre 0,6 % en poids et 5,0 % en poids.

2. Procédé selon la revendication 1, dans lequel l'oxydation du glycérol est effectuée à la température de 50 à 70 °C à la pression atmosphérique.

3. Procédé selon la revendication 1, dans lequel l'oxydation du glycérol est effectuée à la température de 50 à 70 °C à une pression comprise entre 20 265 et 101 325 Pa (entre 0,2 et 1,0 atm), la concentration molaire initiale en glycérol c₀(glyc) est sélectionnée dans la plage comprise entre 0,3 et 1,0 mol/L (M) ; la concentration molaire initiale en hydroxyde de sodium c₀(NaOH) est sélectionnée dans la plage comprise entre 0,7 et 2,0 mol/L (M), le rapport molaire de glycérol et de platine n(glyc)/n(Pt) est compris entre 200 et 500 mol/mol.

4. Procédé selon l'une quelconque des revendications précédentes, dans lequel le temps d'imprégnation du support est sélectionné dans la plage comprise entre 5 et 120 minutes.

5. Procédé selon l'une quelconque des revendications précédentes, dans lequel la pyrolyse est effectuée à une température dans la plage comprise entre 300 et 500 °C pendant 5 à 120 minutes à pression atmosphérique.
